# EUROPEAN PATENT SPECIFICATION

(11) **EP 3 485 860 B1**
(45) Date of publication and mention of the grant of the patent: **27.05.2020**
(21) Application number: 17846089.5
(22) Date of filing: 09.08.2017
(51) Int. Cl.: A61F 13/494, A61F 13/475

(54) **ABSORBENT ARTICLE**
SAUGFÄHIGER ARTIKEL
ARTICLE ABSORBANT

(30) Priority: 02.09.2016 JP 2016171714
(43) Date of publication of application: 22.05.2019
(73) Proprietor: Unicharm Corporation, Ehime 799-0111 (JP)
(72) Inventor: MUKAI, Hirotomo, Kanonji-shi Kagawa 769-1602 (JP)
(74) Representative: Dolleymores
(86) International application number: PCT/JP2017/028947
(87) International publication number: WO 2018/043080

(56) References cited:
- EP-A2- 1 208 826
- JP-A- H09 313 532
- JP-A- H11 514 551
- JP-A- 2008 055 110
- JP-A- 2008 055 110
- JP-A- 2012 192 058

## Description

### [Technical Field]

The present invention relates to an absorbent article.

### [Background Art]

There have been conventionally known absorbent articles, such as pull-on disposable diapers, including leak-proof walls that are raised upright from both sides of an absorbent body toward the skin side of a wearer. In general, in order to prevent excrement that has been absorbed by an absorbent body from leaking outside of the absorbent article, a leak-proof wall is given with contractibility by being provided with an elastic member (elastic strings) which improves fit around wearer's legs. When arranging elastic members on a leak-proof wall, the following method is common: sheet members (nonwoven fabrics) constituting the leak-proof wall are overlaid, making the elastic member in an extended state sandwiched therebetween. For example, PTL 1 discloses an example of arranging an elastic member in a method in which a sheet member is folded such that the elastic member is sandwiched therebetween and the sheet member is welded (embossed) at a plurality of positions.

### [Citation List]

### [Patent Literature]

[PTL 1] Japanese Patent Application Publication No. 2016-93423. Further prior art arrangements are known from JP2008055110 and EP1208826.

### [Summary of Invention]

### [Technical Problem]

In PTL 1, the strength of the leak-proof wall s increased by forming many welded parts all over the sheet member overlaid in the leak-proof wall. However, the leak-proof wall is a portion that is directly in contact with a wearer's groin when the absorbent article is put on, and thus there is a risk that an excessively high strength degrades the texture and fit thereof and causes leakage of excrement or the like. In particular, in the case where the sheet member constituting the leak-proof wall is made of a nonwoven fabric (e.g., a spun-bond nonwoven fabric), the nonwoven fabric is pre-embossed in some cases. In such a case, there is a risk that the originally-embossed parts may overlap welded parts where the overlaid nonwoven fabrics are welded, further degrading the texture of the leak-proof wall.

The present invention was achieved in light of conventional problems such as that described above, and an aspect of the present invention is to suppress degradation of the texture of an absorbent article with leak-proof walls.

### [Solution to Problem]

A main aspect of the present invention for achieving the above-described aspect is an absorbent article as recited by Claim 1.

Features of the present invention other than the above will become clear by reading the description of the present specification with reference to the accompanying drawings.

### [Advantageous Effects of Invention]

According to the present invention, it is possible to suppress degradation of the texture of an absorbent article with leak-proof walls.

### [Brief Description of the Drawings]

[FIG. 1] FIG. 1 is a schematic perspective view when a diaper 1 is seen from a front side.
[FIG. 2] FIG. 2 is a plan view of the diaper 1 that is in an unfolded and extended state.
[FIG. 3] FIG. 3A is a schematic cross sectional view of FIG. 2 taken along the line A-A. FIG. 3B is a schematic cross sectional view of FIG. 2 taken along the line B-B.
[FIG. 4] FIG. 4 is a diagram illustrating first welded parts 61 provided on a surface of a sheet member 50s.
[FIG. 5] FIG. 5 is a diagram illustrating the arrangement of second welded parts 62 provided on an overlaid part 50L.
[FIG. 6] FIG. 6 is a diagram illustrating the details of the second welded parts 62.
[FIG. 7] FIG. 7 is a schematic view illustrating a cross sectional structure in a lateral direction of the overlaid part 50L.
[FIG. 8] FIG. 8 is a schematic cross sectional view illustrating a modification of the structure of the overlaid part 50L of a leak-proof wall 50.

### [Description of Embodiments]

At least the following matters will become clear with the description of this specification and the attached drawings. An absorbent article including:
an absorbent body having a longitudinal direction and a lateral direction orthogonal to each other; and
a pair of leak-proof walls provided on both lateral sides of the absorbent body,
   each of the leak-proof walls formed of a sheet member including a plurality of first welded parts on a surface of the sheet member ,
   each of the leak-proof walls including an overlaid part where the sheet member is overlaid in a thickness direction such that an elastic member stretching/contracting in the longitudinal direction is sandwiched,
   a plurality of second welded parts that join the overlaid sheet member disposed in at least a part of a region of the overlaid part,
   an area of the second welded parts being smaller than an area of the first welded parts,
   wherein an average value of thicknesses of portions, in a longitudinal central region of the overlaid part, where the second welded parts are respectively formed
   is smaller than
   an average value of thicknesses of portions, in a longitudinal end region of the overlaid part, where the second welded parts are respectively formed, and
   an average value of thicknesses of portions, in a longitudinal central region of the overlaid part, where the second welded parts are respectively formed
   is smaller than
   an average value of thicknesses of portions, in a longitudinal end region of the overlaid part, where the second welded parts are respectively formed.

According to such an absorbent article, it is possible to reduce the probability of forming the first welded part and the second welded part overlaying on each other. This makes it possible to suppress degradation of texture and decrease in welding strength of the second welded part in a portion where the second welded part overlaps the first welded part; the degradation and the decrease are caused by a high rigidity of the overlapping portion where the first welded part and the second welded part overlap. Accordingly, it is possible to suppress degradation of the texture of the leak-proof walls when the absorbent article is put on, as well as, suppress poor fit due to peeling of the overlaid part of the leak-proof walls.

Also, the welding strength of the second welded parts is sufficiently strong, making it easier, in the overlaid part, to suppress the sheet member from peeling or to suppress a wall elastic member from separating. Further, even if the first welded part and the second welded part is formed overlapping, it is possible to compress the sheet member by the second welded part over the first welded part, making it possible to firmly weld the overlaid sheet member.

Further, the welding strength in the longitudinal central region can be made stronger than the welding strength in the longitudinal end region. In this way, even if the area ratio of the second welded parts is low in the longitudinal central region, the welding strength over the entire longitudinal central region is not low, making it possible to suppress the overlaid sheet member from peeling or curling.

In such an absorbent article, it is desirable that
in the overlaid part, the sheet member is folded from one lateral side to another lateral side, overlaying the sheet member in the thickness direction.

According to such an absorbent article, the sheet member folded such that the elastic member is sandwiched between the sheet member is joined by the second welded parts in an overlaying state in the thickness direction. This makes it possible to effectively suppress curing or peeling of the end of the folded sheet member. Accordingly, the leak-proof walls are stably formed, making it easier to suppress degradation of texture or fit thereof.

In such an absorbent article, it is desirable that
the second welded parts are provided on the other lateral side with respect to the elastic member.

According to such an absorbent article, the elastic member interposed in the sheet member that is folded toward the other lateral side (lateral outside) can be suppressed from separating from the other lateral side. Further, the folded sheet member is firmly joined by the second welded parts, around the other lateral end portion. This makes it possible to suppress the sheet member from peeling or curling in the overlaid part.

In such an absorbent article, it is desirable that
a ratio of an area of the second welded parts provided per unit area of the overlaid part
is smaller than
a ratio of an area of the first welded parts provided per unit area of the overlaid part.

According to such an absorbent article, it is possible to further reduce the probability of forming the first welded parts and the second welded parts overlaying on each other. This makes it possible to efficiently form the leak-proof walls having a preferable texture.

In such an absorbent article, it is desirable that
a ratio of an area of the second welded parts provided in a region of a predetermined size in a longitudinal central region of the overlaid part
   is smaller than
a ratio of an area of the second welded parts provided in a region that has a size equal to the predetermined size in a longitudinal end region of the overlaid part.

According to such an absorbent article, the longitudinal end region is a region located near a wearer's waist when the absorbent article is put on, and this can increase the joining strength of the second welded parts so that the joint of the overlaid part will not peel even when the wearer largely moves the wearer's body. In contrast, the longitudinal central region is a region located on the crotch part of a wearer when the absorbent article is put on. Accordingly, making the ratio of a region where the second welded parts are formed smaller prevents the rigidity of the overlaid part from being excessively high, suppressing degradation of the texture at the groin region.

In such an absorbent article, it is desirable that
the second welded parts are arranged side-by-side at a predetermined interval from one side to another side along the longitudinal direction, forming a dot row,
in the overlaid part,
   a lateral distance between the elastic member disposed laterally outermost and the dot row disposed laterally innermost
   is larger than
   a lateral distance between the second welded part disposed laterally outermost and a lateral outer end of the overlaid part.

According to such an absorbent article, increasing the lateral distance (W50i) between the laterally-outermost elastic member and the laterally-innermost dot row suppresses the elastic members from being erroneously cut off due to contact of the pressing projections of an embossing roller when the second welded parts are being formed. In addition, decreasing the lateral distance (W50o) between the laterally-outermost second welded part and the lateral outer end of the overlaid part makes it less likely for the lateral outer end of the overlaid part to curl, making it easier to suppress the overlaid sheet member from peeling.

In such an absorbent article, it is desirable that
the sheet member is folded such that, in the overlaid part, a surface of the sheet member on which the first welded parts are formed is inside in the thickness direction.

According to such an absorbent article, the first welded parts are arranged neither on the skin-side surface nor non-skin-side surface of the overlaid part, making the surfaces of the overlaid part smooth. This can make the texture of the leak-proof walls much smoother when the absorbent article is put on.

In such an absorbent article, it is desirable that
in the overlaid part,
a depth of the second welded parts on one side in the thickness direction is different from a depth of the second welded parts on another side in the thickness direction.

According to such an absorbent article, the overlaid sheet member is firmly pressed from one side to the other side in the thickness direction, making it possible to further increase the welding strength of the sheet member by the second welded parts.

### Embodiment

### Basic Configuration of Diaper 1:

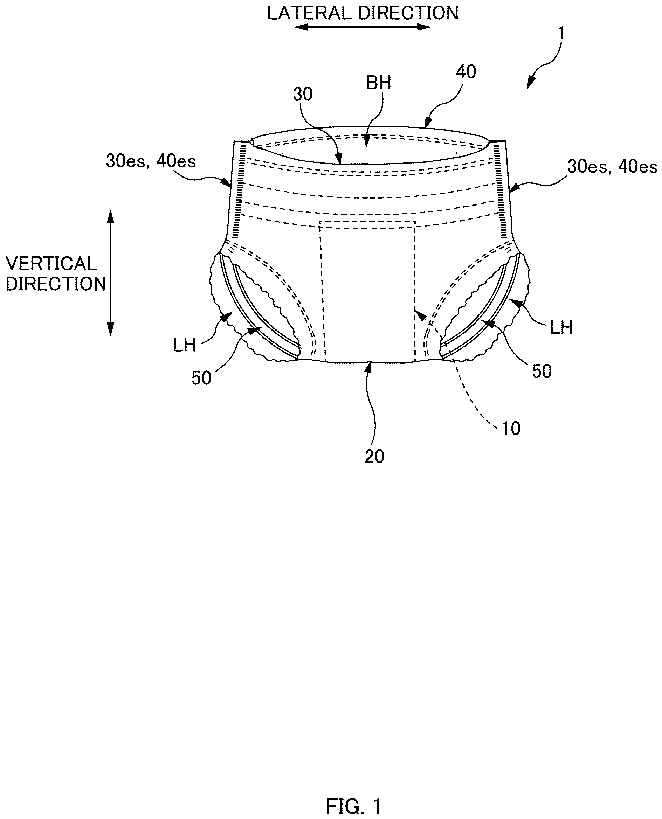

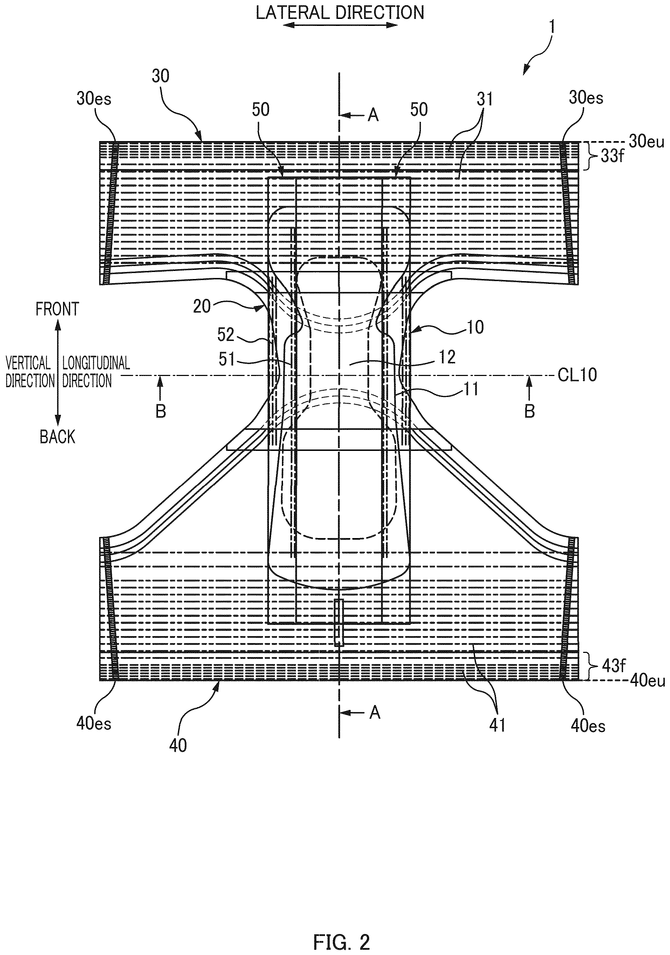

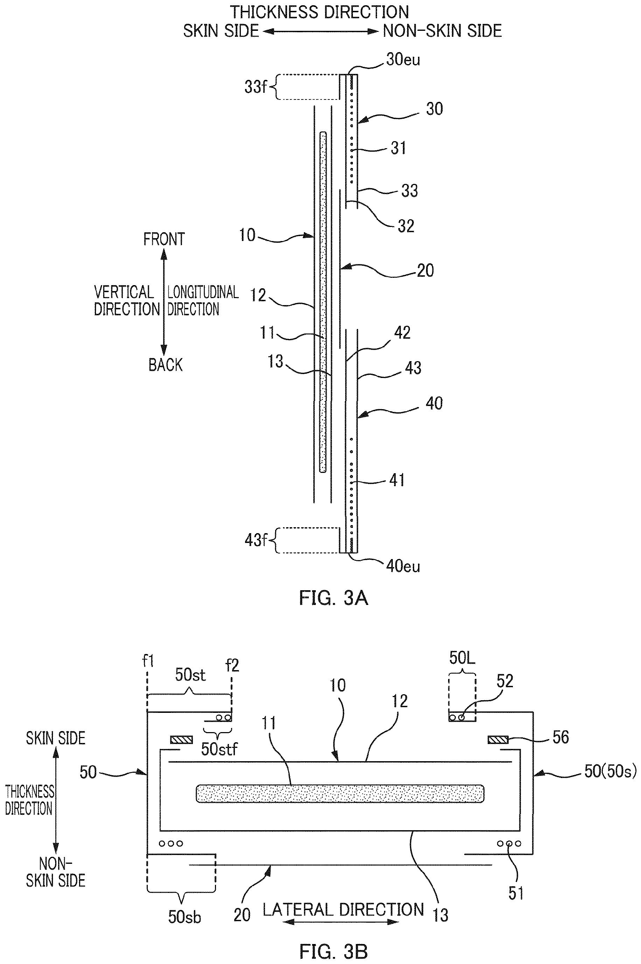

As an example of the absorbent article described in the present embodiment, the basic configuration of a pull-on disposable diaper 1 (hereinafter, also referred to as the "diaper 1") will be described. FIG. 1 is a schematic perspective view when the diaper 1 is seen from the front side. FIG. 2 is a plan view of the diaper 1 that is in an unfolded and extended state. FIG. 3A is a schematic cross sectional view of FIG. 2 taken along the line A-A; and FIG. 3B is a schematic cross sectional view of FIG. 2 taken along the line B-B. Note that the "extended state" in FIG. 2 is a state where the product (the diaper 1) is extended without making creases, specifically, the diaper 1 is extended to the extent that a dimension of each member constituting the diaper 1 (e.g., a later-described front body part 30) becomes the same or almost the same length as the dimension of the member alone.

In the pants-shaped state of FIG. 1, this diaper 1 has three directions orthogonal to one another, namely a vertical direction, a lateral direction, and a front-back direction. Hereinafter, in this pants-shaped state, one side and the other side in the vertical direction are also referred to as the "waist opening side" and the "crotch side, " respectively. And, a front side and a back side in the front-back direction are also referred to as the "front side" and the "back side," respectively.

Whereas, in the unfolded state of FIG. 2, the diaper 1 has three directions orthogonal to one another, namely a longitudinal direction, a lateral direction, and a thickness direction. Hereinafter, in the unfolded state, one side and the other side in the longitudinal direction are also referred to as the "front side" and the "back side," respectively. Note that the above-described lateral direction (equivalent to a width direction) in the unfolded state is the same direction as the lateral direction in the pants-shaped state. The longitudinal direction in the unfolded state is a direction along the vertical direction in the pants-shaped state. Further, as illustrated in FIGS. 3A and 3B, as for the thickness direction orthogonal to the vertical direction (the longitudinal direction) and the lateral direction, the side to be in contact with the skin of a target wearer (a wearer) is referred to as the "skin side" and the opposite side is referred to as the "non-skin side."

The diaper 1 of the present embodiment includes an absorbent main body 10, a crotch exterior part 20, the front body part 30, and a back body part 40. In the diaper 1 which is in the unfolded state of FIG. 2, the absorbent main body 10 is folded into two at a folding position, which is a predetermined longitudinal (vertical) position CL10 of the absorbent main body 10. In this folded state, the front body part 30 and the back body part 40 oppose to each other are joined, and are joined at a front side joint 30es and a back side joint 40es by means such as welding. The front body part 30 and the back body part 40 are thus connected in an annular shape to form a waist opening BH and a pair of leg openings LH, LH as illustrated in FIG. 1, forming the diaper 1 in the pants-shaped state.

The absorbent main body 10 has a function of absorbing excrement, such as urine. The absorbent main body 10 is formed in a substantially rectangle shape in a plan view as illustrated in FIG. 2 and is arranged at the lateral center while the longitudinal direction thereof is arranged along the vertical direction of the diaper 1. The absorbent main body 10 includes a liquid-absorbent, absorbent body 11, a top sheet 12, and a back sheet 13. Leak-proof walls 50 are provided on both lateral sides of the absorbent main body 10. The leak-proof walls 50 will be described later in more detail.

The absorbent body 11 is made by shaping liquid-absorbent fibers (e.g., pulp fibers) in which superabsorbent polymers (SAP) or the like are mixed, into a predetermined shape. Further, a liquid-permeable core wrap sheet that wraps the external peripheral surface of the absorbent body 11 may be provided. The core wrap sheet is, for example, made of a tissue paper or a nonwoven fabric.

The top sheet 12 is a liquid-permeable sheet member, is arranged on the skin-side surface in the thickness direction of the absorbent body 11, and is in contact with the skin of a wearer when the diaper 1 is put on. The top sheet 12 of the present embodiment is formed of, for example, an air-through nonwoven fabric or a spun-bond nonwoven fabric. The back sheet 13 is a liquid-impermeable and moisture-permeable sheet member that is arranged on the non-skin side in the thickness direction of the absorbent body 11. The back sheet 13 is formed of, for example, a resin film. The back sheet 13 prevents moisture (e.g., urine absorbed by the absorbent body 11) from moving (seeping) to the clothing side of the wearer.

The crotch exterior part 20 is an exterior member arranged on the non-skin side of the absorbent main body 10 (the back sheet 13), and is a portion to be located on the crotch part of a wearer when the diaper 1 is put on. In the present embodiment, the crotch exterior part 20 is formed of, for example, a spun-bond nonwoven fabric.

The front body part 30 is an exterior member arranged on the non-skin side of the absorbent main body 10 (the back sheet 13), and is a portion to be located at the front waist part of a wearer when the diaper 1 is put on. The front body part 30 includes an upper exterior sheet 32 and a lower exterior sheet 33 that overlap each other in the thickness direction. As illustrated in FIG. 3A, the upper exterior sheet 32 is arranged on the skin side in the thickness direction, and the lower exterior sheet 33 is arranged on the non-skin side in the thickness direction with respect to the upper exterior sheet 32. The vertical upper end portion of the lower exterior sheet 33 protrudes more vertically upward with respect to the vertical upper end portion of the upper exterior sheet 32. And, the protruded portion is folded back toward the skin side in the thickness direction at the position of a top vertical (longitudinal) end 30eu of the front body part 30, forming a folded part 33f. Since the top end 30eu of the front body part 30 is covered by the folded part 33f, the top end edge of the front body part 30 (i.e., the waist opening BH) is unlikely to bite the skin of a wearer when the diaper 1 is put on, thereby alleviating discomfort around the front waist part.

Further, an elastic member, such as elastic strings, is provided between the upper exterior sheet 32 and the lower exterior sheet 33 in the thickness direction. In the present embodiment, as illustrated in FIGS. 2 and 3A, in a region between the lateral one end portion and the lateral other end portion of the front body part 30, a plurality of front waist elastic members 31 are extended in the lateral direction by a predetermined extension ratio and interposed and joined between the upper exterior sheet 32 and the lower exterior sheet 33. These front waist elastic members 31 provide lateral stretchability to the front body part 30 of the diaper 1.

Note that the "extension ratio" of the elastic member refers to an elongation degree where the natural length of the elastic member (elastic strings) is defined as 1. For example, if the elongation magnification is 1.2, the elastic member is assumed to be extended by 0.2 from the natural length.

The back body part 40 is an exterior member arranged on the non-skin side of the absorbent main body 10 (the back sheet 13) and is a portion to be located at the back waist part of a wearer when the diaper 1 is put on. The structure of the back body part 40 is substantially the same as that of the front body part 30. That is, the back body part 40 includes an upper exterior sheet 42 and a lower exterior sheet 43 that overlap each other in the thickness direction, where the vertical upper end portion of the lower exterior sheet 43 protrudes more vertically upward with respect to the vertical upper end portion of the upper exterior sheet 42. And, the protruded portion is folded back toward the skin side in the thickness direction at the position of a top vertical (longitudinal) end 40eu of the back body part 40, forming a folded part 43f. In this way, the top end edge (the waist opening BH) of the back body part 40 is unlikely to bite the skin of a wearer when the diaper 1 is put on, thereby alleviating discomfort around the back waist part.

As illustrated in FIG. 2, in a region between the lateral one end portion and the lateral other end portion of the back body part 40, a plurality of back waist elastic members 41 are extended in the lateral direction by a predetermined extension ratio and interposed and joined between the upper exterior sheet 42 and the lower exterior sheet 43. This back waist elastic members 41 provide lateral stretchability to the back body part 40 of the diaper 1.

### Leak-proof Wall 50:

The following will describe the leak-proof walls 50. A pair of the leak-proof walls 50 are arranged on both lateral sides of the absorbent main body 10 and extending along the longitudinal direction (the vertical direction of the diaper 1) of the absorbent main body 10. When the diaper 1 is put on, the leak-proof walls 50 are raised upright on both sides of the absorbent main body 10 and suppress excrement from leaking outside of the diaper 1.

In the diaper 1 of the present embodiment, the pair of leak-proof walls 50 are each formed of a sheet member 50s. As the sheet member 50s, for example, a spun-bond nonwoven fabric, a spunbond-meltblown-spunbond (SMS) nonwoven fabric, or the like is used.

As illustrated in FIG. 3B, the one lateral part 50sb of the sheet member 50s is arranged on the non-skin side in the thickness direction of the absorbent main body 10, and is interposed and joined between the back sheet 13 and the crotch exterior part 20. On both lateral sides of the absorbent main body 10, leg elastic members 51 (e.g., elastic strings) that are capable of stretching/contracting in the longitudinal direction (the vertical direction) are provided between the back sheet 13 and the one lateral part 50sb of the sheet member 50s. In the present embodiment, a plurality of (three, in FIG. 3B) leg elastic members 51 are extended in the longitudinal direction by a predetermined extension ratio and interposed and joined between the back sheet 13 and the one lateral part 50sb, providing longitudinal (vertical) stretchability to the sheet member 50s. The region where the leg elastic members 51 are arranged is a portion that is a part to be the leg opening LH of the diaper 1, thereby improving fit of the leg opening LH around the leg of a wearer, when the diaper 1 is put on.

The sheet member 50s is folded laterally from outside to inside at the predetermined lateral position f1, and the other lateral part 50st of the sheet member 50s is joined to the absorbent main body 10 (the top sheet 12) by an adhesive part 56 on the front side in the thickness direction. Note that the "one lateral part" and "the other lateral part" of the sheet member 50s represent "one side" and "the other side" in the lateral direction in a state where the sheet member 50s (the leak-proof wall 50) is extended in the lateral direction (not illustrated) . In the case of FIG. 3B, the "one side" is equivalent to inside in the lateral direction of the sheet member 50s in the unfolded state, while "the other side" is equivalent to outside in the lateral direction of the sheet member 50s in the unfolded state.

Then, the other lateral part 50st of the sheet member 50s is folded from one lateral side to the other lateral side at a predetermined lateral position f2, and the folded portion (referred to as folded part 50stf) is joined to the other lateral part 50st in an overlaying state in the thickness direction (refer to FIG. 3B) . Hereinafter, the portion where the sheet member 50s overlay is also referred to as an overlaid part 50L. The overlaid part 50L is provided with leak-proof-wall elastic members 52 (e.g., elastic strings) that are capable of stretching/contracting in the longitudinal direction (the vertical direction) , between the folded part 50stf and the other lateral part 50st of the sheet member 50s . In the present embodiment, a plurality of (two, in FIG. 3B) leak-proof-wall elastic members 52 are extended in the longitudinal direction by a predetermined extension ratio and interposed and joined between the overlaid part 50L in the thickness direction. Accordingly, stretching force along the longitudinal direction (the vertical direction) is given to the overlaid part 50L. When the diaper 1 is put on, due to the stretching force provided by the leak-proof-wall elastic members 52, the other lateral part 50st of the sheet member 50s (overlaid part 50L) raises upright from the adhesive part 56 (a standing base point) toward the skin side in the thickness direction, thus serving as the leak-proof wall 50. Note that the stretchability of the leak-proof-wall elastic members 52 is applied at least to a longitudinal (vertical) range in FIG. 2 where the leak-proof-wall elastic members 52 overlap the crotch exterior part 20. The longitudinal length of the range where the stretchability of the leak-proof-wall elastic members 52 is applied is the effective length of the leak-proof-wall elastic members 52.

Note that, the overlaid part 50L of the present embodiment is formed by folding one sheet member 50s at the predetermined position f2, thereby overlaying the sheet member 50s. However, the overlaid part 50L may be formed of different members. For example, the overlaid part 50L may be formed of two pieces of sheet members (nonwoven fabric sheets) that are overlaid on each other and joined in the thickness direction. Even in such a case, the leak-proof walls 50 can also raise upright by stretching force of the leak-proof-wall elastic members 52 that are interposed and joined between the sheet members.

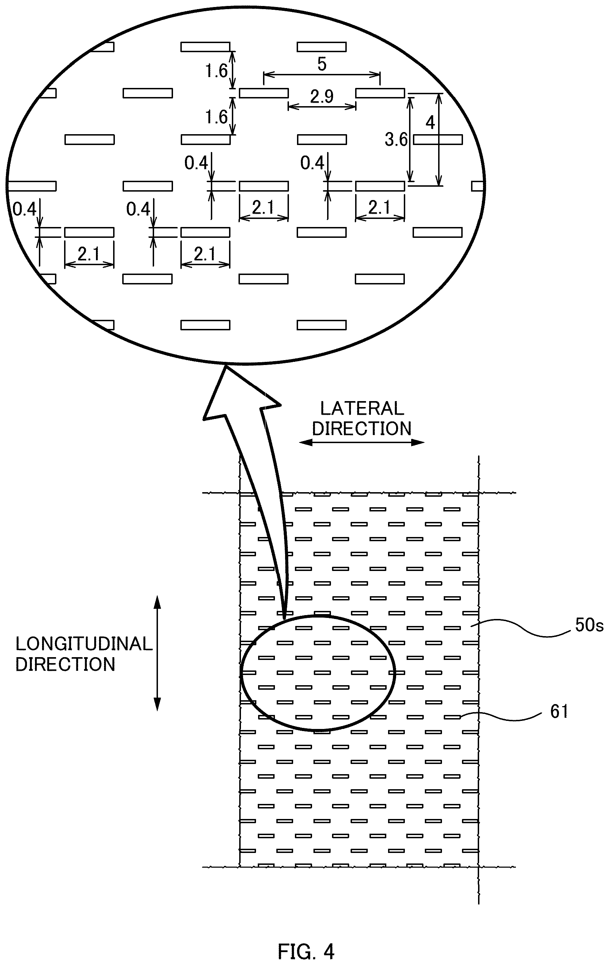

The following will describe the sheet member 50s (a nonwoven fabric) constituting the leak-proof walls 50. As described above, the sheet member 50s of the present embodiment is a spun-bond nonwoven fabric or a SMS nonwoven fabric. On the surface of the nonwoven fabric, a plurality of embosses (welded parts) are provided for maintaining strength. Hereinafter, these embosses are also referred to as the first welded parts 61. FIG. 4 is a diagram illustrating the first welded parts 61 provided on the surface of the sheet member 50s. On the surface of the sheet member 50s, laterally-elongated first welded parts 61 as illustrated in FIG. 4 are regularly formed over the entire area. The pitch of the welded parts is preferably approximately 2 to 6 mm in a vertical direction and in a lateral direction. With the range, the nonwoven fabric can easily maintain flexibility while maintaining strength. Each of the first welded parts 61 in the present embodiment is of a rectangle shape with 2.1 mm in the lateral direction and 0.4 mm in the vertical direction. The first welded parts 61 are arranged in a staggered pattern with a lateral pitch of 5 mm and a vertical pitch of 3. 6 mm. This facilitates achieving both strength and flexibility of the nonwoven fabric.

Further, in FIG. 4, two first welded parts 61 are provided per range of 4 mm x 5 mm. Thus, the ratio of the area of the first welded parts 61 provided per unit area on the sheet member 50s (hereinafter, also referred to as the "area ratio") is (2.1 mm x 0.4 mm) x 2/(4 mm x 5 mm) = 0.084. That is, the area ratio of the first welded parts 61 on the sheet member 50s is 8.4%.

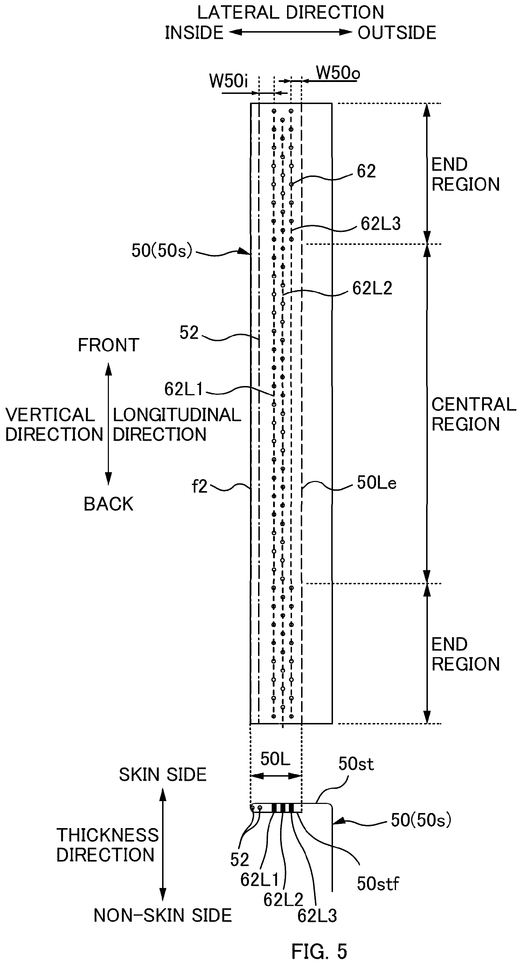

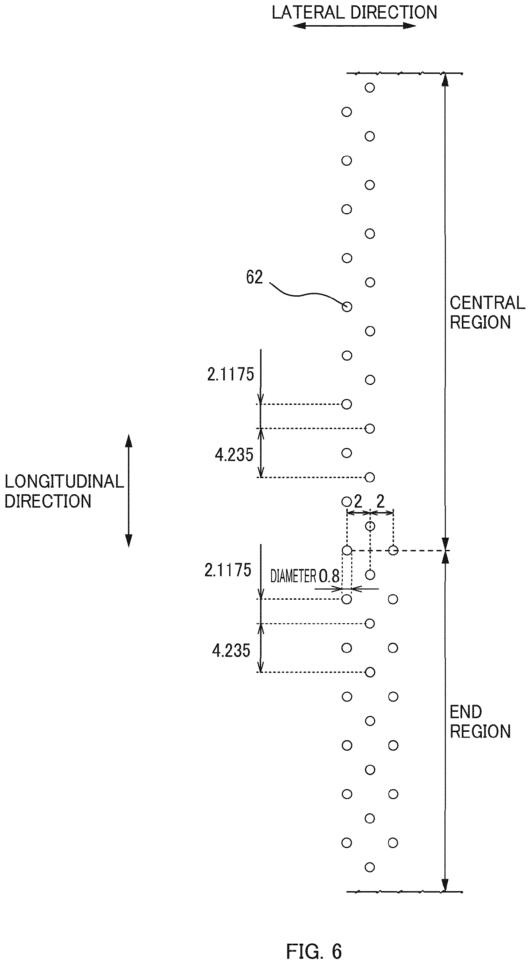

The following will describe the overlaid part 50L of the leak-proof wall 50 in detail. In the overlaid part 50L, the sheet member 50s that is overlaid in the thickness direction is joined by using a welding means such as heat sealing. Hereinafter, welded parts that join the overlaid sheet member 50s are also referred to as second welded parts 62. FIG. 5 is a diagram illustrating the arrangement of the second welded parts 62 provided on the overlaid part 50L. FIG. 6 is a diagram illustrating the details of the second welded parts 62.

As illustrated in FIG. 5, the overlaid part 50L of the leak-proof wall 50 is provided with a plurality of dot-shaped second welded parts 62. The second welded parts 62 are formed outside in the lateral direction with respect to lateral positions where the leak-proof-wall elastic members 52 are arranged. In this way, the leak-proof-wall elastic members 52 that are sandwiched in the thickness direction between the sheet member 50s at the overlaid part 50L can be suppressed from separating from the outside in the lateral direction (lateral outer end 50Le in FIG. 5). Further, since the end portion (the lateral outer end 50Le) of the folded part 50stf is firmly joined to the other lateral part 50st, the folded part 50stf is effectively suppressed from peeling or curling in the overlaid part 50L of the leak-proof wall 50 when the diaper 1 is put on.

The second welded parts 62 are arranged side-by-side at a predetermined interval from one side to the other side along the longitudinal direction (the vertical direction) , forming a dot row 62L. In the present embodiment, a plurality of dot rows 62L are formed side-by-side in the lateral direction at a predetermined interval. Further, the numbers of the dot rows 62L are different between the longitudinal (vertical) central region and the end regions on both sides of the longitudinal central region. Specifically, in the longitudinal central region, two dot rows 62L, namely 62L and 62L2, are formed. On the other hand, in the longitudinal end regions, three dot rows of 62L1 to 62L3 are formed. Note that the "central region" is a region to be arranged on the crotch part of a wearer when the diaper 1 is put on, and is a region that overlaps at least the crotch exterior part 20, in FIG. 2. Whereas, the "end region" is a region including at least a region where stretchability of the leak-proof-wall elastic members 52 is not applied in the longitudinal direction.

In the joining of the overlaid sheet member (the nonwoven fabric) , for maintaining sufficient joining strength, it is preferable that the pitch of the welded parts is approximately 1 to 5 mm in a vertical direction and in a lateral direction. In the present embodiment, as illustrated in FIG. 6, each of the second welded parts 62 that form the dot row 62L is a substantially circle having a diameter of 0.8 mm, and arranged at a longitudinal (vertical) pitch of 4.235 mm. Further, the pitch between dot rows 62L that are adjacent in the lateral direction is 2 mm, and the dots (second welded parts 62) are arranged in a staggered pattern in the adjacent dot rows 62L. This facilitates maintaining sufficient welding strength.

In a range where the second welded parts 62 are formed in the longitudinal central region of the overlaid part 50L, two second welded parts 62 are provided per region of 15 mm x 4.235 mm. Note that 15 mm is the lateral length (width) of the overlaid part 50L, and the region of 15 mm x 4.235 mm is equivalent to a region of the width of 4.235 mm in the longitudinal direction taken out from the overlaid part 50L illustrated as the plan view in FIG. 5. That is, the area ratio of the second welded parts 62 in the longitudinal central region is (0.4 mm × 0.4 mm × 3.14) × 2/(15 mm × 4.235 mm) x 100 = 1. 582%. Similarly, in a range where the second welded parts 62 are formed in each longitudinal end region of the overlaid part 50L, three second welded parts 62 are provided per range of 15 mm × 4.235 mm. That is, the area ratio of the second welded parts 62 in the longitudinal end region is (0.4 mm × 0.4 mm × 3.14) × 3/ (15 mm × 4.235 mm) × 100 = 2.373%.

The longitudinal end regions are regions located near the front side waist and back side waist of a wearer when the diaper 1 is put on. The leak-proof walls 50 are not raised upright in these regions and are in contact with a wearer's body in substantially parallel to the absorbent main body 10 as illustrated in FIG. 3. Thus, on both longitudinal end regions, the second welded parts 62 are formed in a ratio (the area ratio = 2.373%) that can ensure sufficient joining strength so that the joint of the overlaid part 50L will not peel or curl even when a wearer largely moves the wearer's body when the diaper 1 is put on. In contrast, the longitudinal central region is a region located on the crotch part of a wearer when the diaper 1 is put on, and, at the central region, the leak-proof walls 50 are pressed against the wearer's groin. Accordingly, in the central region, making the ratio of the second welded parts 62 being formed as small as possible (the area ratio = 1.582%) prevents the rigidity of the overlaid part 50L of each leak-proof wall 50 from being excessively high, suppressing degradation of the texture at the groin region.

As described above, the plurality of first welded parts 61 are provided in advance on the surface of the sheet member 50s constituting the leak-proof wall 50. Thus, both first welded parts 61 and second welded parts 62 are formed in the overlaid part 50L. When the two types of welded parts, namely the first welded parts 61 and the second welded parts 62, are formed in the same region of the overlaid part 50L, some of the first and second welded parts are formed overlaying on each other in the thickness direction in some cases. For example, if a second welded part is formed overlapping on a portion where a first welded part increases the rigidity of the sheet member, the rigidity of the sheet member at the overlapping portion further increases, which might degrade the texture. In addition, when the two types of welded parts overlap, welding of the second welded part that is formed later may be insufficient, and this is a risk of weakening the joining strength of the overlaid sheet members and of causing the sheet member to peel or curl.

To address this, the diaper 1 of the present embodiment reduces the probability of forming the first welded parts 61 and the second welded parts 62 overlaying on each other by making the area of the second welded parts 62 (0.4 mm × 0.4 mm × 3.14 = 0.502 mm2) smaller, compared to the area of the first welded parts 61 (2.1 mm × 0.4 mm = 0.84 mm2) . In this way, in the diaper 1, it is possible to effectively suppress excessive high rigidity of the leak-proof walls 50 or poor welding, making it possible to form the leak-proof walls 50 with good texture. In conventional diapers, welded parts corresponding to the second welded parts 62 are formed with a large size for ensuring welding of overlaid sheet members, and as a result, the first welded parts 61 and the second welded parts 62 are likely to overlap each other, making more likely to degrade the texture of the overlapping portion. However, the diaper 1 according to the present embodiment can solve such a problem.

Furthermore, comparing the area ratios of the welded parts in the overlaid part 50L, the area ratio for the first welded parts 61 is 8.4% whereas the second welded parts 62 is 1.582 to 2.373%. That is, in the overlaid part 50L, the ratio of the second welded parts 62 formed per unit area is not more than half the ratio of the first welded parts 61 formed per unit area. Accordingly, in the present embodiment, the probability that the first welded parts 61 and the second welded parts 62 overlap further decreases, making it possible to more effectively form the leak-proof walls 50 which has preferable texture and whose overlaid part 50L is less likely to peel.

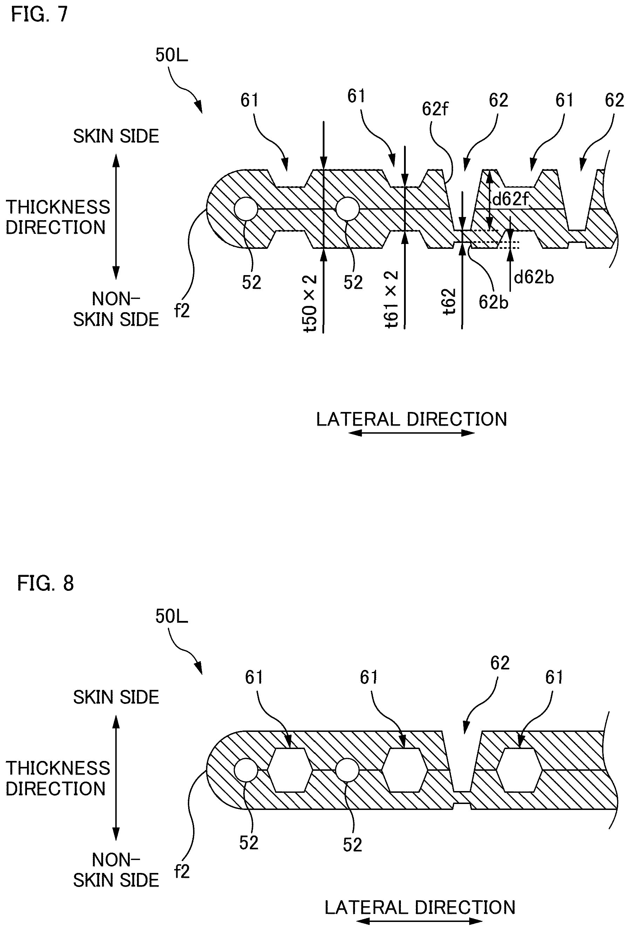

The following will focus on the thickness of portions of the overlaid part 50L where the first welded parts 61 and the second welded parts 62 are formed. FIG. 7 is a schematic view illustrating a cross sectional structure in the lateral direction of the overlaid part 50L. As illustrated in FIG. 7, in the overlaid part 50L, the sheet member 50s (indicated by the shaded part in FIG. 7) is folded at the predetermined lateral position f2 and is overlaid in the thickness direction so that the leak-proof-wall elastic members 52 are sandwiched therebetween. On the surface of the overlaid part 50L, the plurality of first welded parts 61 and the plurality of second welded parts 62 are formed. Note that, in the example of FIG. 7, for simplification of description, illustrated is a case where there are arranged at an identical lateral position the first welded parts 61 formed on the skin-side surface of the overlaid part 50L and the first welded parts 61 formed on the non-skin-side surface of the overlaid part 50L. However, the first welded parts 61 on the skin side may be located at positions different from the first welded parts 61 on the non-skin side. Further, in FIG. 7, the lateral widths of the first welded parts 61 and the second welded parts 62 are depicted with different scales, for convenience of description. The actual sizes of the welded parts 61 and 62 have been described with reference to FIGS. 4 and 6.

In FIG. 7, when the thickness of the sheet member 50s is denoted by t50, and the thickness of a portion where the first welded part 61 is formed is denoted by t61 (t50 > t61), the thickness of the overlaid part 50L is represented as t50 × 2, and the minimum value of the thickness of the portion where the first welded parts 61 are formed in the overlaid part is t61 × 2. Note that "the minimum value of the thickness of the portion where the first welded part 61 is formed" refers to the thickness of the overlaid part 50L when the lateral positions of the first welded parts 61 on the skin-side surface overlap the lateral positions of the first welded parts 61 on the non-skin-side surface as illustrated in FIG. 7. Further, the "thickness" is the average value of the results (n = 10) of measuring a plurality of positions (e.g., 10 positions) within a measurement range of the overlaid part 50L by using a thickness measuring instrument (e.g., Keyence Digital Micro Scope VHX-2000) .

In FIG. 7, the thickness of a portion where the second welded part 62 is formed is denoted by t62. In order to firmly join the sheet member 50s which is overlaid in two-layer, the thickness t62 of the portion where the second welded part 62 is formed is welded to the extent that the thickness t62 becomes thinner than the thickness t61 × 2 of a portion where the first welded part 61 is formed (t61 × 2 > t62). That is, increasing the compressing force for forming the second welded parts 62 improves the welding strength of the second welded parts. This makes it easier, in the overlaid part 50L, to suppress the sheet member 50s from peeling and to suppress the leak-proof-wall elastic members 52 from separating. Further, even when the first welded part 61 and the second welded part 62 are formed overlapping, it is possible to form the second welded part 62 by further compressing the first welded part 61 as long as the relationship of t61 × 2 > t62 is maintained. This makes it possible to more securely weld the overlaid sheet member 50.

Note that, as illustrated in FIG. 7, the second welded part 62 includes a recess 62f formed on the skin-side surface and a recess 62b formed on the non-skin-side surface. And the depth d62f of the recess 62f is larger than the depth d62b of the recess 62b. Here, the "depth" refers to a distance in the thickness direction between the surface (skin-side surface or non-skin-side surface) of the sheet member 50s and the bottom part of the second welded part 62 (refer to FIG. 7) .

When forming the second welded parts 62, while the sheet member 50s overlaid in the thickness direction is transported in the direction of transport along the longitudinal direction, welding such as ultrasonic welding or heat welding is performed, for example, using a projecting pattern provided on the outer circumferential surface of an embossing roller whose rotation axis extends along the lateral direction. In the case of FIG. 7, the embossing roller applies a pressure to the skin-side surface in the thickness direction, making larger the depth of the recess 62f formed on the skin-side surface. When the pressure applied by the embossing roller is released, the restoring force where the bottom part of the recess 62f that has been pressed deep toward the non-skin side in the thickness direction returns to the skin side is exerted. Consequently, a shallower recess 62b is formed on the non-skin-side surface of the recess 62f. Accordingly, as illustrated in FIG. 7, each second welded part 62 has a structure in which recesses 62f and 62b are respectively formed on the skin side and on the non-skin side in the thickness direction.

In this way, the second welded part 62 is formed such that the recess (the recess 62f) on one side in the thickness direction becomes deeper than the recess (the recess 62b) on the other side, this enables the overlaid sheet member 50s to be firmly pressed from the one side to the other side in the thickness direction, increasing welding strength of the sheet member. In the present embodiment, since the area of the second welded parts 62 is smaller than the area of the first welded parts 61, there is a risk that the welding strength of the second welded parts 62 is insufficient. Accordingly, strong welding of the second welded parts 62 such that the recesses 62f (the recesses 62b) become as deep as possible makes it possible to ensure sufficient welding strength of the sheet member 50s in the overlaid part 50L.

Further, in the diaper 1, the depth d62fc (not illustrated) of the recess 62f of each second welded part 62 formed in the longitudinal (vertical) central region of FIG. 5 is larger than the depth d62fe (not illustrated) of the recess 62f of each second welded part 62 formed in the end regions in the longitudinal direction (d62fc > d62fe). As described above, the second welded parts 62 are formed by being applied with a pressure in the thickness direction by the embossing roller while the sheet member 50s (the leak-proof walls 50) is transported in the direction of transport. On the outer circumferential surface of the embossing roller, formed alternately are a region where three rows of projecting patterns are provided side-by-side in the lateral direction and a region where two rows of projecting patterns are provided side-by-side in the lateral direction. Pressing the three rows of projecting patterns against forms three rows of dot rows 62L1 to 62L3 in the longitudinal end regions of the overlaid part 50L. On the other hand, pressing the two rows of projecting patterns against forms two rows of dot rows 62L and 62L2 in the longitudinal central region of the overlaid part 50L.

The pressing force (that is, the compression force in the thickness direction) of the embossing roller against the overlaid part 50L is constant through the pressing process, and therefore a pressure applied on each projecting pattern is larger in a portion with two rows of projecting patterns than in a portion with three rows of projecting patterns. Accordingly, the depth d62fc of the second welded parts 62 forming two dot rows 62L1 and 62L2 in the longitudinal central region is deeper than the depth d62fe of the second welded parts 62 forming three dot rows 62L1 to 62L3 in the longitudinal end regions, increasing welding strength. In this way, even if the area ratio of the second welded parts 62 is low in the longitudinal central region (refer to FIG. 5), it is possible to suppress the sheet member 50s that is overlaid in the overlaid part 50L from peeling or curling. Note that, even if the depth d62fe of the recess 62f in the longitudinal end regions are different from the depth d62fc of the recess 62f in the longitudinal central region, the area of the second welded parts 62 are equal. Consequently, the problem of degrading texture is unlikely to occur by deepening the depth d62fc of the recess 62f in the longitudinal central region.

As illustrated in FIG. 5, in the diaper 1, a lateral distance between a laterally-outermost one of the plurality of leak-proof-wall elastic members 52 provided in the overlaid part 50L and a laterally-innermost one (the dot row 62L1) of the plurality of second welded parts 62 is defined as a lateral distance W50i, and a lateral distance between a laterally-outermost one (the dot row 62L3) of the plurality of second welded parts 62 provided on the overlaid part 50L and the lateral outer end 50Le of the overlaid part 50L is defined as a lateral distance W50o. The lateral distance W50i is larger than the lateral distance W50o. Increasing the distance W50i makes it less likely for the projecting pattern of the embossing roller to touch the leak-proof-wall elastic members 52 when forming the second welded parts 62 (the dot row 62L1) . This suppresses the leak-proof-wall elastic members 52 from being cut off erroneously. In contrast, decreasing the distance W50o makes it less likely for the lateral outer end 50Le of the overlaid part 50L to curl, making it easier to suppress the overlaid sheet member 50s from peeling.

### Modification:

FIG. 8 is a schematic cross sectional view illustrating a modification of the structure of the overlaid part 50L of the leak-proof wall 50, and FIG. 8 is a diagram corresponding to FIG. 7. In the example of FIG. 7, the plurality of first welded parts 61 are positioned outside in the thickness direction. That is, the sheet member 50s is folded at the predetermined position f2 so that, in the thickness direction, the first welded parts 61 are arranged on the skin-side surface and on the non-skin-side surface. In contrast, in a modification, the overlaid part 50L is formed by folding the sheet member 50s such that the plurality of first welded parts 61 are positioned inside in the thickness direction. In other words, the sheet member 50s is folded such that the surface of the sheet member 50s where the first welded parts 61 are formed is inside in the thickness direction. That is, in the overlaid part 50L of the modification, the first welded parts 61 are interposed inside in the thickness direction.

In this way, only the second welded parts 62 are arranged on the surface of the overlaid part 50L, and the surface of the overlaid part 50L is formed smooth compared with the case of FIG. 7. This can make the texture of the leak-proof walls 50 much smoother when the diaper 1 is put on.

### Other embodiments

Although the embodiment of the present disclosure have been described hereinabove, the above embodiment of the present disclosure are simply to facilitate understanding of the present disclosure and are not in any way to be construed as limiting the present disclosure. The present disclosure may variously be changed or altered without departing from its gist and encompass equivalents thereof. For example, modification which will be described below is possible.

Although, an example where elastic strings are used for the waist elastic members and the leak-proof-wall elastic members has been described in the above embodiment, these elastic members are not limited to linear elastic members such as elastic strings. For example, a planer (band) elastic member with a predetermined width may instead be used. Further, the following configuration is also acceptable: sheet members having stretchability (e.g., stretchable nonwoven fabric) are used for the sheet members constituting the front body part 30 and the back body part 40, making it unnecessary to separately include elastic members such as elastic strings.

### [Reference Signs List]

- 1:: Diaper (Pull-on disposable diaper), Absorbent article;
- 10:: Absorbent main body;
- 11:: Absorbent body;
- 12:: top sheet;
- 13:: Back sheet;
- 20:: crotch exterior part;
- 30:: Front body part;
- 30es:: front side joint;
- 30eu:: top end;
- 31:: front waist elastic member;
- 32:: upper exterior sheet;
- 33:: lower exterior sheet;
- 33f:: folded part;
- 40:: Back body part;
- 40es:: Back side-joint;
- 40eu:: top end;
- 41:: back waist elastic member;
- 42:: upper exterior sheet;
- 43:: lower exterior sheet;
- 43f:: folded part;
- 50:: Leak-proof wall;
- 50s:: Sheet member;
- 50sb:: one lateral part;
- 50st:: other lateral part;
- 50stf:: folded part;
- 50L:: Overlaid part;
- 51:: Leg elastic member;
- 52:: leak-proof-wall elastic member;
- 56:: Adhesive part;
- 61:: First welded part;
- 62:: Second welded part;
- 62L:: Dot row;
- 62L1 to 62L3:: Dot row;
- 62f:: Recess;
- 62b:: Recess;
- f1, f2:: Predetermined position;
- t50:: Thickness (Sheet member 50s);
- t61:: Thickness (First welded part 61);
- t62:: Thickness (Second welded part 62);
- W50i:: Distance;
- W50o:: Distance;
- BH:: Waist opening;
- LH:: leg opening;
- CL10:: Predetermined position.

## Claims

1. An absorbent article (1) comprising:
an absorbent body (10) having a longitudinal direction and a lateral direction orthogonal to each other; and
a pair of leak-proof walls (50) provided on both lateral sides of the absorbent body,
each of the leak-proof walls formed of a sheet member (50s) including a plurality of first welded parts (61) on a surface of the sheet member,
**characterised by** each of the leak-proof walls including an overlaid part (50L) where the sheet member is overlaid in a thickness direction such that an elastic member (52) stretching/contracting in the longitudinal direction is sandwiched,
a plurality of second welded parts (62) that join the overlaid sheet member disposed in at least a part of a region of the overlaid part,
an area of the second welded parts being smaller than an area of the first welded parts,
wherein an average value of thicknesses of portions, in the overlaid part, where the first welded parts are respectively formed is larger than an average value of thicknesses of portions, in the overlaid part, where the second welded parts are respectively formed, and
an average value of thicknesses of portions, in a longitudinal central region of the overlaid part, where the second welded parts are respectively formed is smaller than an average value of thicknesses of portions, in a longitudinal end region of the overlaid part, where the second welded parts are respectively formed.

2. The absorbent article according to claim 1, wherein
in the overlaid part, the sheet member is folded from one lateral side to another lateral side, overlaying the sheet member in the thickness direction.

3. The absorbent article according to claim 2, wherein
the second welded parts are provided on the other lateral side with respect to the elastic member.

4. The absorbent article according to any one of claims 1 to 3, wherein
a ratio of an area of the second welded parts provided per unit area of the overlaid part
is smaller than
a ratio of an area of the first welded parts provided per unit area of the overlaid part.

5. The absorbent article according to claim 4, wherein
a ratio of an area of the second welded parts provided in a region of a predetermined size in a longitudinal central region of the overlaid part
is smaller than
a ratio of an area of the second welded parts provided in a region that has a size equal to the predetermined size in a longitudinal end region of the overlaid part.

6. The absorbent article according to any one of claims 1 to 5, wherein
the second welded parts are arranged side-by-side at a predetermined interval from one side to another side along the longitudinal direction, forming a dot row,
in the overlaid part,
a lateral distance between the elastic member disposed laterally outermost and the dot row disposed laterally innermost
is larger than
a lateral distance between the second welded part disposed laterally outermost and a lateral outer end of the overlaid part.

7. The absorbent article according to any one of claims 1 to 6, wherein
the sheet member is folded such that, in the overlaid part, a surface of the sheet member on which the first welded parts are formed is inside in the thickness direction.

8. The absorbent article according to any one of claims 1 to 7, wherein
in the overlaid part,
a depth of the second welded parts on one side in the thickness direction is different from a depth of the second welded parts on another side in the thickness direction.

## Patentansprüche

1. Absorbierender Artikel (1), der Folgendes umfasst:
einen Saugkörper (10), der eine Längsrichtung und eine laterale Richtung, orthogonal zueinander, aufweist; und
ein Paar von auslaufsicheren Wänden (50), die an beiden lateralen Seiten des Saugkörpers bereitgestellt sind,
wobei jede der auslaufsicheren Wände, die aus einem Lagenelement (50s) ausgebildet sind, eine Vielzahl von ersten verschweißten Teilen (61) auf einer Oberfläche des Lagenelements einschließt,
**dadurch gekennzeichnet, dass** jede der auslaufsicheren Wände ein überlagertes Teil (50L) einschließt, wo das Lagenelement in einer Dickenrichtung überlagert ist, sodass ein elastisches Element (52), das sich in der Längsrichtung dehnt/zusammenzieht, eingeschoben ist,
eine Vielzahl von zweiten verschweißten Teilen (62), die das überlagerte Lagenelement verbinden, in zumindest einem Teil eines Bereichs des überlagerten Teils angeordnet ist,
eine Fläche der zweiten verschweißten Teile kleiner ist als eine Fläche der ersten verschweißten Teile,
wobei ein Durchschnittswert der Dicken der Abschnitte in dem überlagerten Teil, wo die ersten verschweißten Teile jeweils ausgebildet sind, größer ist als ein Durchschnittswert der Dicken der Abschnitte in dem überlagerten Teil, wo die zweiten verschweißten Teile jeweils ausgebildet sind, und
ein Durchschnittswert der Dicken der Abschnitte in einem längsgerichteten mittleren Bereich des überlagerten Teils, wo die zweiten verschweißten Teile jeweils ausgebildet sind, kleiner ist als ein Durchschnittswert der Dicken der Abschnitte in einem längsgerichteten Endbereich des überlagerten Teils, wo die zweiten verschweißten Teile jeweils ausgebildet sind.

2. Absorbierender Artikel nach Anspruch 1, wobei in dem überlagerten Teil das Lagenelement von einer lateralen Seite zu einer anderen lateralen Seite gefaltet ist, wobei das Lagenelement in der Dickenrichtung überlagert.

3. Absorbierender Artikel nach Anspruch 2, wobei
die zweiten verschweißten Teile auf der anderen lateralen Seite, in Bezug auf das elastische Element, bereitgestellt sind.

4. Absorbierender Artikel nach einem der Ansprüche 1 bis 3, wobei
ein Anteil einer Fläche der zweiten verschweißten Teile, die pro Einheitsfläche des überlagerten Teils bereitgestellt ist,
kleiner ist als
ein Anteil einer Fläche der ersten verschweißten Teile, die pro Einheitsfläche des überlagerten Teils bereitgestellt ist.

5. Absorbierender Artikel nach Anspruch 4, wobei
ein Anteil einer Fläche der zweiten verschweißten Teile, die in einem Bereich einer vorgegebenen Größe in einem längsgerichteten mittleren Bereich des überlagerten Teils bereitgestellt ist,
kleiner ist als
ein Anteil einer Fläche der zweiten verschweißten Teile, die in einem Bereich bereitgestellt ist, der eine Größe aufweist, die gleich der vorgegebenen Größe in einem längsgerichteten Endbereich des überlagerten Teils ist.

6. Absorbierender Artikel nach einem der Ansprüche 1 bis 5, wobei
die zweiten verschweißten Teile nebeneinander in einem vorgegebenen Abstand von einer Seite zu einer anderen Seite entlang der Längsrichtung in dem überlagerten Teil angeordnet sind, wobei eine Punktzeile ausgebildet wird,
wobei eine laterale Entfernung zwischen dem elastischen Element, das lateral am äußersten angeordnet ist, und der Punktzeile, die lateral am innersten angeordnet ist,
größer ist als
eine laterale Entfernung zwischen dem zweiten verschweißten Teil, das lateral am äußersten angeordnet ist, und einem lateralen Außenende des überlagerten Teils.

7. Absorbierender Artikel nach einem der Ansprüche 1 bis 6, wobei
das Lagenelement derart gefaltet ist, dass in dem überlagerten Teil eine Oberfläche des Lagenelements, auf der die ersten verschweißten Teile ausgebildet sind, innen in der Dickenrichtung vorliegt.

8. Absorbierender Artikel nach einem der Ansprüche 1 bis 7, wobei in dem überlagerten Teil
sich eine Tiefe der zweiten verschweißten Teile auf einer Seite in der Dickenrichtung von einer Tiefe der zweiten verschweißten Teile auf einer anderen Seite der Dickenrichtung unterscheidet.

## Revendications

1. Article absorbant (1) comportant :
un corps absorbant (10) ayant une direction allant dans le sens longitudinal et une direction allant dans le sens latéral qui sont orthogonales l'une par rapport à l'autre ; et
une paire de parois résistantes aux fuites (50) mises en oeuvre sur les deux côtés latéraux du corps absorbant,
chacune des parois résistantes aux fuites formées à partir d'un élément de feuille (50s) comprenant une pluralité de premières parties soudées (61) sur une surface de l'élément de feuille,
**caractérisé par** chacune des parois résistantes aux fuites comprenant une partie recouverte (50L) comme quoi l'élément de feuille est recouvert dans une direction allant dans le sens de l'épaisseur de telle sorte qu'un élément élastique (52) s'étirant/se contractant dans la direction allant dans le sens longitudinal est pris en sandwich,
une pluralité de deuxièmes parties soudées (62) qui raccordent l'élément de feuille recouvert se trouvant dans au moins une partie d'une région de la partie recouverte,
une surface des deuxièmes parties soudées étant inférieure par rapport à une surface des premières parties soudées,
dans lequel une valeur moyenne des épaisseurs des parties, dans la partie recouverte, où les premières parties soudées sont formées respectivement, est supérieure par rapport à une valeur moyenne des épaisseurs des parties, dans la partie recouverte, où les deuxièmes parties soudées sont formées respectivement, et
une valeur moyenne des épaisseurs des parties, dans une région centrale longitudinale de la partie recouverte, où les deuxièmes parties soudées sont formées respectivement, est inférieure par rapport à une valeur moyenne des épaisseurs des parties, dans une région d'extrémité longitudinale de la partie recouverte, où les deuxièmes parties soudées sont formées respectivement.

2. Article absorbant selon la revendication 1, dans lequel
dans la partie recouverte, l'élément de feuille est plié depuis un côté latéral jusqu'à un autre côté latéral, pour recouvrir l'élément de feuille dans la direction allant dans le sens de l'épaisseur.

3. Article absorbant selon la revendication 2, dans lequel
les deuxièmes parties soudées sont mises en oeuvre sur l'autre côté latéral par rapport à l'élément élastique.

4. Article absorbant selon l'une quelconque des revendications 1 à 3, dans lequel
un rapport d'une surface des deuxièmes parties soudées fourni par unité de surface de la partie recouverte
est inférieur par rapport à
un rapport d'une surface des premières parties soudées fourni par unité de surface de la partie recouverte.

5. Article absorbant selon la revendication 4, dans lequel
un rapport d'une surface des deuxièmes parties soudées fourni dans une région d'une taille prédéterminée dans une région centrale longitudinale de la partie recouverte
est inférieur par rapport à
un rapport d'une surface des deuxièmes parties soudées fourni dans une région qui a une taille égale à la taille prédéterminée dans une région d'extrémité longitudinale de la partie recouverte.

6. Article absorbant selon l'une quelconque des revendications 1 à 5, dans lequel
les deuxièmes parties soudées sont agencées côte à côte selon un intervalle prédéterminé depuis un côté jusqu'à un autre côté le long de la direction allant dans le sens longitudinal, pour former une rangée de points,
dans la partie recouverte,
une distance latérale entre l'élément élastique se trouvant le plus à l'extérieur dans le sens latéral et la rangée de points se trouvant le plus à l'intérieur dans le sens latéral
est supérieure par rapport à
une distance latérale entre la deuxième partie soudée se trouvant le plus à l'extérieur dans le sens latéral et une extrémité extérieure latérale de la partie recouverte.

7. Article absorbant selon l'une quelconque des revendications 1 à 6, dans lequel
l'élément de feuille est plié de telle sorte que, dans la partie recouverte, une surface de l'élément de feuille sur laquelle les premières parties soudées sont formées se trouve à l'intérieur dans la direction allant dans le sens de l'épaisseur.

8. Article absorbant selon l'une quelconque des revendications 1 à 7, dans lequel dans la partie recouverte,
une profondeur des deuxièmes parties soudées sur un côté dans la direction allant dans le sens de l'épaisseur est différente par rapport à une profondeur des deuxièmes parties soudées sur un autre côté dans la direction allant dans le sens de l'épaisseur.
